# EUROPÄISCHE PATENTSCHRIFT

(11) **EP 2 465 476 B1**
(45) Veröffentlichungstag und Bekanntmachung des Hinweises auf die Patenterteilung: **17.06.2020**
(21) Anmeldenummer: 11191734.0
(22) Anmeldetag: 02.12.2011
(51) Int. Cl.: A61F 2/86

(54) **Stent und Verfahren zu seiner Herstellung**
Stent and method for producing the same
Stent et son procédé de fabrication

(30) Priorität: 15.12.2010 US 423123 P
(43) Veröffentlichungstag der Anmeldung: 20.06.2012
(73) Patentinhaber: Biotronik AG, 8180 Bülach (CH)
(72) Erfinder: Klocke, Björn, 8006 Zürich (CH); Borck, Alexander, 91086 Aurachtal (DE)
(74) Vertreter: Galander, Marcus

(56) Entgegenhaltungen:
- WO-A1-03/037223
- US-A1- 2006 136 048

## Beschreibung

Die vorliegende Erfindung betrifft ein Implantat, insbesondere eine intraluminale Endoprothese, mit einem Körper und mindestens einer auf dem Körper angeordneten abluminalen Schicht, welche vorzugsweise mindestens eine pharmazeutisch aktive Substanz enthält, sowie ein entsprechendes Verfahren zur Herstellung eines derartigen Implantats.

Medizinische Endoprothesen oder Implantate für die unterschiedlichsten Anwendungen sind in großer Vielfalt aus dem Stand der Technik bekannt. Als Implantate im Sinne der vorliegenden Erfindung werden insbesondere endovaskuläre Prothesen, beispielsweise Stents, betrachtet.

In einer heutzutage häufig angewendeten Therapie, insbesondere bei Herz-Kreislauf-Erkrankungen, werden Stents eingesetzt. Diese dienen dazu, Hohlorgane durchgängig zu halten. Sie weisen häufig einen Körper in der Form eines ggf. durchbrochenen rohrförmigen oder hohlzylinderförmigen Grundgitters auf, das an beiden Längsenden offen ist. Das rohrförmige Grundgitter einer derartigen Endoprothese wird in das zu behandelnde Hohlorgan eingesetzt und stützt dort das behandelte Hohlorgan. Stents haben sich insbesondere zur Behandlung von Blutgefäßerkrankungen etabliert. Durch den Einsatz von Stents ggf. unter Zuhilfenahme z.B. eines Ballonkatheters können verengte Bereiche in Blutgefäßen erweitert und offen gehalten werden, so dass ein Lumengewinn resultiert. Stents können aber auch beispielsweise bei der Krebsbehandlung dazu dienen, durch bösartige Tumore verursachte Verengungen von Atemwegen (z.B. der Luftröhre), Gallenwegen oder der Speiseröhre nach einer Aufdehnung offen zu halten.

Durch den Einsatz von Stents oder anderen Implantaten kann zwar ein für den Therapieerfolg primär notwendiger optimaler Querschnitt des Hohlorgans erreicht werden, allerdings initiiert die dauerhafte Anwesenheit eines derartigen Fremdkörpers eine Kaskade von mikrobiologischen Prozessen, die beispielsweise eine Entzündung (Inflammation) des behandelten Hohlorgans oder eine nekrotische Veränderung begünstigen und die zu einem allmählichen Zuwachsen des Stents durch Bildung von Plaques führen können. Im schlimmsten Fall kann diese Veränderung des Hohlorgans einen Verschluss des Hohlorgans bedingen.

Es ist wünschenswert, dass die oben beschriebene, eine Inflammation begünstigende Wirkung von Implantaten in Zukunft weitestgehend vermieden wird, da hierdurch die Wirksamkeit des Implantats verringert wird und weitere Schädigungen des behandelten Organismus hervorgerufen werden können.

Um die Zellneubildung zu hemmen, werden bereits sogenannte Drug Eluting Stents (DES) eingesetzt, welche kleine Menge vor Arzneistoffen wie Sirolimus und Paclitaxel freigeben. Solche DES werden insbesondere zur Therapie der koronaren Herzkrankheit - insbesondere bei einem erhöhten Risiko einer Restenose (beispielsweise bei Diabetikern) - eingesetzt.

Weiter hat sich gezeigt, dass abluminal beschichtete DES konzeptionell komplett beschichteten DES überlegen sind, da sie ein verbessertes Einwachsverhalten zeigen. Hierbei enthält die abluminale Schicht die pharmazeutisch aktive Substanz wie z.B. Sirolimus und Paclitaxel. Jedoch ergaben Tests von abluminal beschichteten Stents, dass die Schichthaftung der abluminalen Schicht schlecht ist und zu regulatorischen, produktionsbezogenen und gegebenenfalls auch klinischen Problemen führen kann.

Um die Schichthaftung zu verbessern, wurde bisher der Werkstoff Parylene als Haftvermittler oder eine Plasmabehandlung getestet. Mit diesen Methoden wurde die Haftung der abluminalen Beschichtung jedoch nicht wesentlich verbessert.

Aus der Druckschrift US 2008/0206440 A1 ist ein bioabsorbierbarer Stent mit Stegen bekannt, welche jeweils abluminal eine pharmazeutische Zusammensetzung aufweisen, die durch eine Beschichtung eingekapselt ist, in deren Matrix Antikörper enthalten sind. Die in der Antikörper-Beschichtung enthaltenen Antikörper oder Antikörperfragmente dienen dazu, Antigene des Hohlorgans zu binden, welche für das behandelte Individuum spezifisch sind. Hierdurch wird das Einwachsverhalten des Stents in die jeweiligen Gefäßzellen begünstigt. In WO03/037223 A1 und US 2006/0136048 A1 werden ebenfalls beschichtete Stents beschrieben.

In der Druckschrift US 2008/0097575 A1 wird eine medizinische Einrichtung mit einer Beschichtung beschrieben. Die Beschichtung ist so gestaltet, dass einerseits eine luminale oder mit dem Blut in Berührung kommende Oberfläche eines Stents oder Stent-Grafts vorgesehen ist und andererseits eine äußere Oberfläche realisiert ist, welche daran angepasst ist, den Kontakt zwischen dem angrenzendem Gewebe und dem Stent oder Stent-Graft zu verbessern. Auch diese Gestaltung eines Stents oder Stent-Grafts enthält keine Lösung dahingehend, eine bessere Haftung der abluminalen Schicht zu erreichen.

Folglich besteht die Aufgabe der vorliegenden Erfindung darin zu verhindern, dass eine abluminale Schicht von der Oberfläche des Implantatkörpers gelöst wird. Die Aufgabe besteht ferner darin, die abluminale Schicht möglichst einfach gegen Abheben und Abscheren zu schützen. Entsprechend besteht die Aufgabe der Erfindung darin, ein solches Implantat zu schaffen und ein einfaches Verfahren zur Herstellung eines solchen Implantats anzugeben.

Die obige Aufgabenstellung wird gelöst durch das in Anspruch 1 angegebene erfindungsgemäße Implantat.

Es wird insbesondere vorgeschlagen, dass die mindestens eine abluminale Schicht in einem Übergangsbereich mittels mindestens einer, auf der abluminalen Schicht angeordneten Verankerungsschicht zusätzlich an dem Implantatkörpers befestigt ist, wobei die Verankerungsschicht die abluminale Schicht teilweise, nämlich im Übergangsbereich, bedeckt.

Durch die Verwendung einer zusätzlichen Verankerungsschicht, welche vorzugsweise degradierbar ist, wird eine bessere Haftung der abluminalen Schicht in dem Übergangsbereich Implantatkörper ermöglicht.

Als Übergangsbereich oder Übergangszone wird ein Endbereich der abluminalen Schicht bezeichnet, welcher am weitesten in luminaler Richtung angeordnet ist. Durch die Anordnung der Verankerungsschicht im Übergangsbereich werden auf der abluminalen Schicht mögliche Angriffspunkte für die Abheben oder das Abscheren der abluminalen Schicht durch die Verankerungsschicht bedeckt und somit verankert, so dass ein Ablösen der abluminalen Schicht bereits von vorn herein verhindert wird. Es ist von Vorteil, wenn die Verankerungsschicht die abluminale Schicht lediglich im Übergangsbereich überlappt oder überdeckt, da sonst die Freisetzung einer pharmazeutisch aktiven Substanz aus der abluminale Schicht nicht sofort nach Einsetzen in das behandelte Hohlorgan erfolgen kann, weil die Verankerungsschicht eine Barriere darstellen würde.

Vorzugsweise beträgt die von der Verankerungsschicht abgedeckte Oberfläche der abluminalen Schicht im Übergangsbereich weniger als 30% der gesamten Oberfläche der abluminalen Schicht.

Die Verankerungsschicht kann als Degradationsschicht durch mechanische Strukturierung, Beimischung oder Oberflächenmodifikation besonders endothelialisierungs-freundlich gestaltet werden, um die Einheilung des Implantats zu fördern. Insbesondere kann das Einwachsverhalten eines Implantats, insbesondere eines Stents, durch die Morphologie seiner Oberfläche beschleunigt werden. Beispielsweise begünstigen bestimmte periodische Strukturen, wie z.B. Linien, mit Periodizitätslängen im Bereich 0,1 µm bis 30 µm (lateral) vorteilhaft die Ansiedelung von Endothelzellen. Die Tiefe der Strukturen kann dabei im Bereich von 200 nm bis 5 µm liegen. Ein positiver Einfluss auf die Reendothelialisierung wird auch erreicht, wenn Antikörper, wie beispielsweise Anti-CD34, auf der Oberfläche der Verankerungsschicht immobilisiert werden. Ebenso können RGD Peptidsequenzen auf der Oberfläche der Verankerungsschicht immobilisiert werden. Diese Peptidketten binden an Integrine. Dadurch wird eine Signalkaskade angestoßen, die zur Migration und anschließender Proliferation von Zielzellen führt.

Der Vorteil der erfindungsgemäßen Lösung besteht weiter darin, dass die anfangs erwähnten technischen, regulatorischen und klinischen Probleme reduziert bzw. abgestellt werden. Die in der abluminalen Schicht vorzugsweise enthaltenen antiproliferativen Wirkstoffe werden nur gewebeseitig (abluminal) freigesetzt und entfalten somit genau dort ihre Wirkung, wo ihr Bestimmungsort ist. Darüber hinaus kann die luminale Seite des Implantats durch geschickte Wahl der Zusammensetzung der Verankerungsschicht optimal an die luminalen Gegebenheiten des Hohlorgans angepasst werden, in das das Implantat eingesetzt ist. Zusätzlich kann daher das Einwachsverhalten des Implantats auf der luminalen Seite - verglichen mit einer glatten Metalloberfläche - verbessert werden. Demgegenüber ist die Endothelbedeckung bei einem komplett mit einer Medikamentenbeschichtung versehenen Implantat auf der luminalen Seite limitiert.

Aufgrund der beschleunigten Einheilung des erfindungsgemäßen Implantats und der aufgrund der endothelialisierungs-freundlichen Verankerungsschicht möglichen Reduktion von Einwachshemmern wird es in vorteilhafter Weise möglich, die Dual-Anti-Platelet-Therapie zu verkürzen.

Bei der vorliegenden Erfindung umfasst der Körper des Implantats mindestens einen Teil des Implantats, vorzugsweise den Hauptteil des Implantats, welcher die mechanische Integrität des Implantats bewirkt.

Wie bereits oben ausgeführt wurde, ist es von Vorteil, wenn die mindestens eine Verankerungsschicht degradierbar ist. Vorzugsweise ist die mindestens eine Verankerungsschicht derart ausgebildet, dass sie in vivo schneller als die mindestens eine abluminale Schicht degradiert. Die schnell degradierbare Verankerungsschicht wird nach dem Implantieren (und ggf. nach dem Einwachsen) des Implantats in Bezug auf ihre mechanische Haltefunktion nicht mehr benötigt. Eine schnelle Degradation der Verankerungsschicht ist daher zwangsläufig vorteilhaft, da Fremdkörper in einem Organ generell zu vermeiden sind und die Verankerungsschicht im Übergangsbereich die mit der pharmazeutisch aktiven Substanz versehene abluminale Schicht bedeckt. Insofern sind Degradationszeiten zwischen typischerweise einem Tag und 6 Monaten vorteilhaft.

Die mindestens eine Verankerungsschicht erstreckt sich in den luminalen Bereich des Implantats. Luminal werden die abluminal vorgesehenen pharmazeutisch aktiven Substanzen nicht benötigt, so dass sie in diesem Bereich überflüssig oder sogar schädlich sind. Gegebenenfalls können in der Verankerungsschicht andere, sich von den Wirkstoffen der abluminalen Schicht unterscheidende pharmazeutisch aktive Substanzen eingesetzt werden.

In einer Weiterbildung der vorliegenden Erfindung ist zwischen der mindestens einen abluminalen Schicht und der mindestens einen Verankerungsschicht im Übergangsbereich ein Stoffschluss und/oder Formschluss ausgebildet. Beispielsweise wird die abluminale Schicht in dem Übergangsbereich zunächst durch ein Lösungsmittel, in welchem das Material der Verankerungsschicht gelöst ist, angelöst. Dies wird insbesondere auch dadurch erreicht, dass die Verankerungsschicht die abluminale Schicht in dem Übergangsbereich überlappt. Ggf. kann die abluminale Schicht auch zwischen einer ersten und einer zweiten Verankerungsschicht derart eingebettet sein, dass die Schichten im Übergangsbereich überlappen. Bei dieser Anordnung ist es von Vorteil, wenn in dem Übergangsbereich n abluminale Schichten zwischen zwei der n+1 Verankerungsschichten angeordnet, wobei n>=1 und wobei n eine ganze Zahl ist. In diesem Fall ist die Verankerung der abluminalen Schicht besonders gut. Alternativ ist auch die Verwendung von lediglich n Verankerungsschichten möglich, wobei in diesem Fall die erste, direkt auf der Oberfläche des Körpers angeordnete abluminale Schicht im Übergangsbereich nicht zwischen zwei Verankerungsschichten angeordnet ist, sondern im Übergangsbereich lediglich von einer Verankerungsschicht bedeckt ist.

Bevorzugt werden, um die Degradierbarkeit der Verankerungsschicht herzustellen, als Material der Verankerungsschicht Polymere, z.B. kurzkettiges PLGA (Polylactid-co-Glycolid) oder P4HB (Poly-4-Hydroxybutyrat), eingesetzt. Ebenfalls von Vorteil können eine Aufprägung eines eine Oberflächenstruktur ausbildenden Musters und/oder eine Kombination mit einem chemischen Endothel-Förderer (z.B. zyklische Integrin-bindende Arg-Gly-Asp Peptide, cRGD) sein. Alternativ kann für abluminale Schicht und Verankerungsschicht die gleiche Polymer-Matrix verwendet werden, wobei die abluminale Schicht zusätzlich mindestens eine pharmazeutisch aktive Substanz aufweist.

Um ebenfalls ein weiter verbessertes Einwachsverhalten des Implantats zu erreichen, wird auf der Oberfläche der außen liegenden mindestens einen Verankerungsschicht zumindest teilweise eine weitere Beschichtung angeordnet, welche vorzugsweise einen Endothel-Fänger ("Pro-Healing") und/oder einen Endothelialisierungs-Promotor enthält und/oder eine endothelialisierungsfördernde Oberfläche aufweist.

Für die abluminale Schicht und die Verankerungsschicht geeignete Werkstoffe können beispielsweise Polymere und/oder Metalle enthalten. Die Schichten können dabei aus mehreren dieser Werkstoffe bestehen. Gemeinsames Merkmal dieser Werkstoffe ist ihre Biodegradierbarkeit. Beispiele für geeignete polymere Verbindungen sind Polymere aus der Gruppe Cellulose, Kollagen, Albumin, Casein, Polysaccharide (PSAC), Polylactid (PLA), Poly-L-Lactid (PLLA), Polyglykol (PGA), Poly-D,L-Lactid-co-glycolid (PDLLA-PGA), Polyhydroxybuttersäure (PHB), Polyhydroxyvaleriansäure (PHV), Polyalkylcarbonate, Polyorthoester, Polyethylenterephtalat (PET), Polymalonsäure (PML), Polyanhydride, Polyphosphazene, Polyaminosäuren und deren Copolymere sowie Hyaluronsäure. Die Polymere können je nach den gewünschten Eigenschaften in Reinform, in derivatisierter Form, in Form von Blends oder als Copolymere vorliegen. Metallische biodegradierbare Werkstoffe basieren vorwiegend auf Legierungen von Magnesium, Mangan, Wolfram und Eisen.

Unter Biodegradation werden chemische, insbesondere hydrolytische, enzymatische und andere stoffwechselbedingte Abbauprozesse im lebenden Organismus verstanden, die vor allem durch die mit dem biodegradierbaren Werkstoff des Implantats in Kontakt gelangenden Körperflüssigkeiten verursacht werden und zu einer allmählichen Auflösung der den biodegradierbaren Werkstoff enthaltenden Strukturen des Implantats führen. Das Implantat verliert durch diesen Prozess zu einem bestimmten Zeitpunkt seine mechanische Integrität. Synonym zu dem Begriff Biodegradation wird häufig der Begriff Biokorrosion verwendet. Der Begriff Bioresorption umfasst die anschließende Resorption der Abbauprodukte durch den lebenden Organismus.

Vorteilhafterweise enthalten die abluminale Schicht(en) und die Verankerungsschicht(en) ein degradierbares Polymer oder bestehen aus einem solchen Polymer. Es ist denkbar, dass die Verankerungsschicht(en) (i) schneller, (ii) langsamer oder (iii) gleich schnell wie die abluminale Schicht degradiert. Dabei wird die Degradationsgeschwindigkeit über die Molmasse und die Wahl des Polymers gesteuert, vorteilhafterweise in der Größenordnung von 1 Woche bis 6 Monaten. Je nach Indikation macht (i) Sinn, wenn die abluminale Schicht auch nach der Implantation noch gegen Verrutschen fixiert werden muss. Variante (ii) ist dann vorteilhaft, wenn die Einheilung bzw. Endothelialisierung durch eine schnelle Freilegung der Oberfläche des Implantatkörpers forciert werden soll. Schließlich beinhaltet die Möglichkeit (iii) Vorteile, wenn z.B. im Rahmen einer Prozessvereinfachung mit den gleichen Polymeren gearbeitet werden soll.

Die obige Aufgabenstellung wird ferner gelöst durch ein Verfahren umfassend die folgenden Schritte:
a) Bereitstellen des Körpers des Implantats,
b) Aufbringen der mindestens einen Verankerungsschicht auf mindestens einen Übergangsbereich der abluminalen Schicht.

Hierbei wird die Verankerungsschicht derart aufgebracht, dass die Verankerungsschicht die abluminale Schicht teilweise, nämlich in dem Übergangsbereich, bedeckt. Das erfindungsgemäße Verfahren ist besonders einfach, kostengünstig und führt dazu, die abluminale Schicht gegen Abheben und Abscheren zu schützen.

Als besonders vorteilhafte und einfache Verfahren haben sich zum Aufbringen der Verankerungsschicht Sprühverfahren, vorzugsweise mit Abschattung, oder Pipettierverfahren erwiesen.

Merkmale weiterer Ausführungsbeispiele des erfindungsgemäßen Verfahrens wurden bereits oben in Bezug auf das erfindungsgemäße Implantat erläutert. Die entsprechenden Vorteile wurden ebenfalls angegeben und gelten auch für das erfindungsgemäße Verfahren.

Das erfindungsgemäße Verfahren bzw. das erfindungsgemäße Implantat wird nachfolgend in Beispielen anhand von Figuren erläutert. Dabei bilden alle beschriebenen und/oder bildlich dargestellten Merkmale den Gegenstand der Erfindung, auch unabhängig von ihrer Zusammenfassung in den Ansprüchen oder deren Rückbezüge.

Es zeigen schematisch:
- Fig. 1 bis 3: Ausführungsbeispiele für ein erfindungsgemäßes Implantat, veranschaulicht anhand eines Querschnitts einer Stentstrebe und
- Fig. 4 bis 6: drei Ausführungsbeispiel zum Aufbringen der Verankerungsschicht im Rahmen des erfindungsgemäßen Verfahrens.

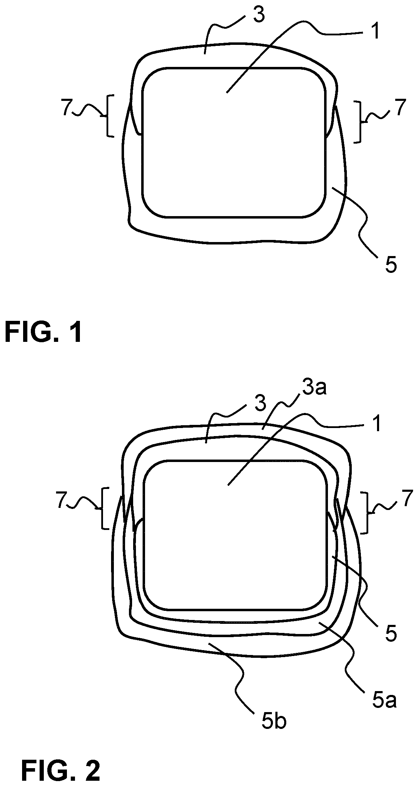

Figur 1 zeigt den Querschnitt einer Strebe 1 eines Stents 10. Die Stentstreben können aus allen handelsüblichen permanenten Werkstoffen hergestellt werden, wie z.B. CoCr oder 316L. In einer besonderen Ausführungsform kann die Strebe auch ganz oder teilweise aus einer biodegradierbarer Metalllegierung (Magnesium, Eisen, Zink, Wolfram, Mangan) oder einem biodegradierbaren Polymer bestehen. Ein insgesamt hohlzylinderförmiges Gitter aus Streben 1 bildet den Körper des Stents 10. Auf der abluminalen Seite der Strebe 1 ist ihrer Oberfläche die abluminale Schicht 3 angeordnet, welche beispielsweise eine Matrix aus PLLA (L210; Boehringer Ingelheim) aufweist. Die Matrix enthält die pharmazeutisch aktive Substanz Rapamycin mit einem Feststoffgehalt von 17% bis 20% Gew.% und einer Flächenbeladung von 1,4 µg/mm².

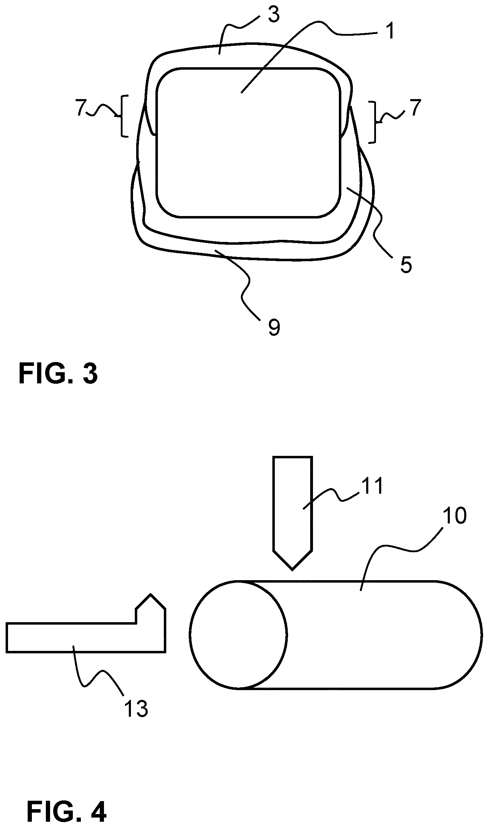

Die abluminale Schicht 3 kann beispielsweise mit der in Figur 4 gezeigten Anordnung hergestellt werden.

Figur 4 veranschaulicht die Außenkontur des Stents 10. Das hohlzylinderförmige Gitter des Stents 10 wird zur Herstellung der abluminalen Schicht 3 auf einen in Figur 4 nicht dargestellten Träger montiert, welcher die luminale Seite des Stents 10 abschattet. Auf der abluminalen Seite wird das mit der pharmazeutisch aktiven Substanz versehene Polymer, (PLGA; LG 858, Boehringer Ingelheim) gelöst in Chloroform (Konzentration 1g/l) mit 17% Gew.% Rapamycin bezogen auf den Feststoffanteil, mittels einer abluminal angeordneten, in radialer Richtung auf den Stent 10 gerichteten Pipettierdüse 11 aufgetragen. Ein Vorteil dieses Verfahrens besteht darin, dass der Stent 10 von allen Seiten beschichtet werden kann.

Vor dem Pipettieren wird der Stent 10, nachdem er auf den zylinderförmigen Träger gesteckt wurde, per Video vermessen. Anschließend wird eine motorgesteuerte Pipettierdüse 11 basierend auf der Videoaufnahme derart gesteuert, dass die gesamte äußere, abluminale Oberfläche des Stents 10 abgefahren und die gewünschte Mischung aus pharmazeutisch aktiver Substanz, Polymer und Lösungsmittel abluminal zur Herstellung der abluminalen Schicht 3 aufgetragen wird. Alternativ ist die manuelle Pipettierung oder auch ein Drehen des Stentkörpers beim Auftragen der abluminalen Schicht 3 möglich.

Nach dem Trocknen der abluminalen Schicht 3 erfolgt die luminale Beschichtung. Dazu wird eine Polymerlösung aus PLLA (L210; Boeringer Ingelheim) in Chloroform hergestellt. Die Polymerkonzentration beträgt dazu 1g/l. Mittels einer weiteren, in radialer Richtung nach außen zeigenden Pipettierdüse 13 wird nun die luminale Verankerungsschicht 5 aufgetragen. Hierbei wird die Pipettierdüse 13 in den inneren Hohlraum des hohlzylinderförmigen Gitters des Stents 10 eingeführt.

In dem Übergangsbereich 7, in dem die abluminale Schicht 3 in luminaler Richtung endet, wird die abluminale Schicht 3 durch das Lösungsmittel der Verankerungsschicht 5 angelöst. Das Material der Verankerungsschicht 5 ist dort mit dem angelösten Übergangsbereich 7 der abluminalen Schicht 3 verbunden. Hierdurch wird im Übergangsbereich 7 eine besonders feste Kopplung der abluminalen Schicht 3 und der Verankerungsschicht 5 erreicht, sodass die abluminale Schicht 3 im Übergangsbereich 7 besonders stark mechanisch befestigt ist. Hierdurch ist die abluminale Schicht 3 besser gegen Abheben und Abscheren geschützt.

Um die Einheilung des Stents in das Gewebe zu fördern, kann die Verankerungsschicht 5 mit mechanischen Strukturen, Zusätzen oder Oberflächenmodifikationen versehen werden. Eine solche Möglichkeit ist in Figur 3 dargestellt. Dort ist auf der Verankerungsschicht 5 eine weitere Schicht 9 angeordnet, welche Endothel-Fänger aufweist. Die Schicht 9 besteht dabei aus LG 858 oder L210 und kann vorzugsweise durch ein Prägewerkzeug mit einer Strukturierung, beispielsweise einer Linienstruktur, versehen werden. Hierbei sind insbesondere Linienstrukturen geeignet, die ein Gittermuster auf der Oberfläche bilden. Allgemein wird unter einer Struktur eine Vertiefung in der Oberfläche verstanden. Bei einer Gitterstruktur sind Vertiefungen in der Form von Linien ausgebildet, welche in einer Richtung parallel zueinander verlaufen und eine erste Schar bilden. Diese erste Schar von periodischen Vertiefungen wird unter einem Winkel von anderen, ebenfalls parallel zueinander verlaufenden Linien (zweite Schar) geschnitten. Bevorzugt ist ein Schnittwinkel von 90° zwischen erster Schar und zweiter Schar.

Zur Herstellung der Strukturen, insbesondere von Feinstrukturen, sind dem Fachmann Prägetechniken prinzipiell bekannt. Alternativ kann die geprägt oder ungeprägte Polymerschicht 9 aus Chitosan bestehen. Das Chitosan (KitoZyme; KIOM.CSU) wird in einer 0,3%-igen Essigsäure gelöst und auf die Oberfläche gesprüht. Die Amingruppen des Chitosans erlauben polymeranaloge Kopplungsreaktionen insbesondere mit Isothocyanatgruppen. So können Biomoleküle, die über eine entsprechende Ankergruppe verfügen, im wässrigen Medium bei Raumtemperatur immobilisiert werden.

Figur 2 zeigt eine erfindungsgemäße Anordnung von zwei abluminalen Schichten 3,3a und drei Verankerungsschichten 5,5a und 5b auf der Oberfläche der Strebe 1. Jeweils eine abluminale Schicht 3,3a ist derart angeordnet, dass diese im Übergangsbereich 7 zwischen jeweils zwei Verankerungsschichten 5 und 5a bzw. 5a und 5b befestigt ist. Hierdurch wird ein besonders guter Schutz der abluminalen Schichten 3,3a gegen Abscheren und Abheben realisiert, insbesondere auch bei der Dilatation des Stents 10 in dem behandelten Hohlorgan.

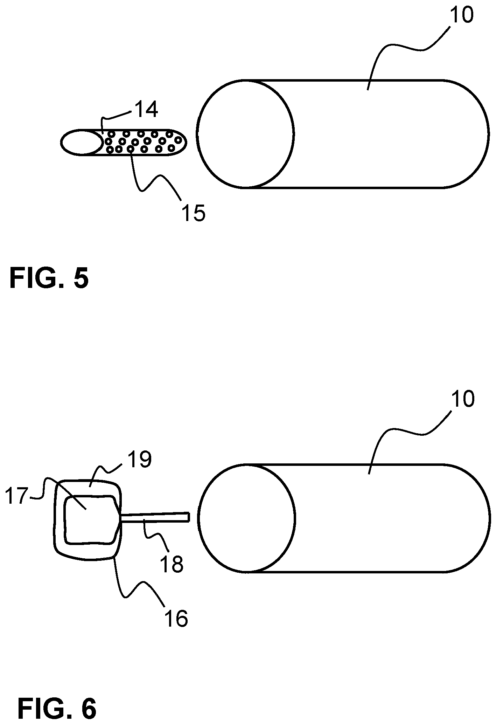

Figur 5 zeigt eine weitere Möglichkeit, die luminal angeordnete Verankerungsschicht 5 aufzubringen. In den Stent 10 wird eine rohrförmige Sprühdüse 14 eingeführt, welche auf ihrem gesamten Umfang über einen vorbestimmten Teil ihrer Länge eine Vielzahl von Öffnungen 15 aufweist, durch die das gelöste Polymer im Wesentlichen in radialer Richtung auf die luminale Seite des Stents 10 gesprüht wird.

Eine weitere Möglichkeit zum Auftragen der Verankerungsschicht 5 ist in Figur 6 dargestellt. Als Mittel zum Auftragen der Verankerungsschicht 5 dient ein Auftragestab 16 mit einem Kopf 17 welcher an einem dünnen Draht oder Stiel 18 befestigt ist. Auf der äußeren Oberfläche des Kopfes 17 ist ein saugfähiges Material 19 angeordnet. Dieses saugfähige Material 19 wird mit der Polymerlösung, beispielsweise PLLA (L210; Boehringer Ingelheim) in Chloroform, getränkt und mit dem Stiel 18 zuerst durch den Stent 10 gezogen. Hierbei ist der Durchmesser des Kopfes 17 mit dem saugfähigen Material 19 des Auftragestabs 16 so gewählt, dass bei Durchziehen des Auftragestabs 16 die Mantelfläche des saugfähigen Materials 19 die luminale Oberfläche des Stents 10 berührt und so die Polymerlösung an der luminalen Oberfläche des Stents 10 abstreift. Nach dem Verdampfen des Lösungsmittels bleibt das unbeladene Polymer an der luminalen Seite des Stents 10 zurück. Das Lösungsmittel Chloroform hat jedoch, z.B. bei dem in Figur 1 dargestellten Ausführungsbeispiel, in dem Übergangsbereich 7 die abluminale Schicht 3 angelöst und eine stabile Verbindung zwischen abluminaler Schicht 3 und luminaler Verankerungsschicht 5 erzeugt. Der so erhaltene Formschluss führt zu einer Verbesserung der mechanischen Eigenschaften. Insbesondere wurden Delaminierungen der abluminalen Schicht 3 nicht mehr beobachtet.

### Bezugszeichenliste

- 1: Strebe eines Stents
- 3, 3a: abluminale Schicht
- 5, 5a, 5b: Verankerungsschicht
- 7: Übergangsbereich
- 9: weitere Schicht
- 10: Stent
- 11: Pipettierdüse zum Aufbringen der abluminalen Schicht 3
- 13: Pipettierdüse zum Aufbringen der Verankerungsschicht 5
- 14: rohrförmige Sprühdüse
- 15: Öffnung
- 16: Auftragestab
- 17: Kopf
- 18: Stiel
- 19: saugfähige Schicht

## Patentansprüche

1. Intraluminale Endoprothese mit einem Körper und mindestens einer auf dem Körper angeordneten abluminalen Schicht (3), welche vorzugsweise mindestens eine pharmazeutisch aktive Substanz enthält, und welche mindestens in einem Übergangsbereich (7), der einen Endbereich der abluminalen Schicht (3) bezeichnet, welcher am weitesten in luminaler Richtung angeordnet ist, mittels mindestens einer auf der abluminalen Schicht (3) angeordneten Verankerungsschicht (5) zusätzlich an dem Implantatkörper befestigt ist, wobei die Verankerungsschicht (5) die abluminale Schicht (3) lediglich im Übergangsbereich überdeckt,
**dadurch gekennzeichnet, dass** sich die mindestens eine Verankerungsschicht (5) in den luminalen Bereich der Endoprothese (10) erstreckt.

2. Endoprothese nach Anspruch 1, **dadurch gekennzeichnet, dass** die von der Verankerungsschicht abgedeckte Oberfläche der abluminalen Schicht im Übergangsbereich weniger als 30% der gesamten Oberfläche der abluminalen Schicht beträgt.

3. Endoprothese nach einem der vorhergehenden Ansprüche, **dadurch gekennzeichnet, dass** die mindestens eine Verankerungsschicht (5) degradierbar ist.

4. Endoprothese nach einem der vorhergehenden Ansprüche, **dadurch gekennzeichnet, dass** die abluminale Schicht (3) degradierbar ist.

5. Endoprothese nach einem der vorhergehenden Ansprüche, **dadurch gekennzeichnet, dass** zwischen der mindestens einen abluminalen Schicht (3) und der mindestens einen Verankerungsschicht (5) im Übergangsbereich (7) ein Stoffschluss und/oder Formschluss ausgebildet ist.

6. Endoprothese nach einem der vorhergehenden Ansprüche, **dadurch gekennzeichnet, dass** n abluminale Schichten (3, 3a) in dem Übergangsbereich (7) zwischen zwei der n+1 Verankerungsschichten (5, 5a, 5b) angeordnet sind, wobei n >= 1 und wobei n eine ganze Zahl ist.

7. Endoprothese nach einem der vorhergehenden Ansprüche, **dadurch gekennzeichnet, dass** die mindestens eine Verankerungsschicht (5) derart ausgebildet ist, dass sie in vivo schneller als die mindestens eine abluminale Schicht (3) degradiert.

8. Endoprothese nach einem der vorhergehenden Ansprüche, **dadurch gekennzeichnet, dass** auf der Oberfläche der außen liegenden Verankerungsschicht (5, 5b) zumindest teilweise eine weitere Beschichtung (9) angeordnet ist, welche vorzugsweise einen Endothel-Fänger und/oder einen Endothelialisierungs-Promotor enthält und/oder eine endothelialisierungsfördernde Oberfläche aufweist.

9. Verfahren zur Herstellung einer intraluminalen Endoprothese mit einem Körper und mindestens einer auf dem Körper angeordneten abluminalen Schicht (3), welche vorzugsweise mindestens eine pharmazeutisch aktive Substanz enthält, umfassend die folgenden Schritte:
a) Bereitstellen des Körpers der Endoprothese (10),
b) Aufbringen der mindestens einen Verankerungsschicht (5) auf mindestens einen Übergangsbereich (7) der abluminalen Schicht (3), vorzugsweise mittels Aufsprühen und/oder Pipettieren,
c) Aufbringen der mindestens einen Verankerungsschicht (5) derart, dass sich die mindestens eine Verankerungsschicht (5) in den luminalen Bereich der Endoprothese (10) erstreckt.

10. Verfahren nach Anspruch 9, **dadurch gekennzeichnet, dass** die mindestens eine Verankerungsschicht (5) degradierbar ist.

11. Verfahren nach einem der Ansprüche 9 bis 10, **dadurch gekennzeichnet, dass** die Verankerungsschicht (5, 5a, 5b) derart aufgebracht wird, dass n abluminale Schichten (3, 3a) in dem Übergangsbereich (7) zwischen zwei der n+1 Verankerungsschichten (5, 5a, 5b) angeordnet ist, wobei n >= 1 und wobei n eine ganze Zahl ist.

12. Verfahren nach einem der Ansprüche 9 bis 11, **dadurch gekennzeichnet, dass** auf die Oberfläche der außen liegenden Verankerungsschicht (5, 5b) zumindest teilweise eine weitere Beschichtung (9) aufgebracht wird, welche vorzugsweise einen Endothel-Fänger und/oder einen Endothelialisierungs-Promotor enthält und/oder eine endothelialisierungsfördernde Oberfläche aufweist.

## Claims

1. An intraluminal endoprosthesis comprising a body and at least one abluminal layer (3), which is disposed on the body, preferably contains at least one pharmaceutically active substance, and is additionally fastened in a transition region (7), which is understood to be an end region of the abluminal layer (3) that is disposed farthest in the luminal direction, to the implant body by way of at least one anchoring layer (5) disposed on the abluminal layer (3), wherein the anchoring layer (5) covers the abluminal layer (3) merely in the transition region,
**characterised in that** the at least one anchoring layer (5) extends in the luminal region of the endoprosthesis (10).

2. The endoprosthesis according to claim 1,**characterised in that** the surface of the abluminal layer covered by the anchoring layer in the transition region is less than 30% of the total surface of the abluminal layer.

3. The endoprosthesis according to either one of the preceding claims, **characterised in that** the at least one anchoring layer (5) is degradable.

4. The endoprosthesis according to any one of the preceding claims, **characterised in that** the abluminal layer (3) is degradable.

5. The endoprosthesis according to any one of the preceding claims, **characterised in that** the at least one abluminal layer (3) is joined to the at least one anchoring layer (5) in the transition region (7) by a material bond and/or interlocking engagement.

6. The endoprosthesis according to any one of the preceding claims, **characterised in that** n abluminal layers (3, 3a) are disposed in the transition region (7) between two of the n+1 anchoring layers (5, 5a, 5b), with n>=1 and n being an integer.

7. The endoprosthesis according to any one of the preceding claims, **characterised in that** the at least one anchoring layer (5) is designed so that it degrades more quickly in vivo than the at least one abluminal layer (3).

8. The endoprosthesis according to any one of the preceding claims, **characterised in that** a further coating (9) is disposed on at least part of the surface of the outer anchoring layer (5, 5b), the further coating preferably containing an endothelial catcher and/or an endothelialisation promoter and/or comprising an endothelialisation-promoting surface.

9. A method for producing an intraluminal endoprosthesis comprising a body and at least one abluminal layer (3), which is disposed on the body and preferably contains at least one pharmaceutically active substance, the method comprising the following steps:
a) providing the body of the endoprosthesis (10),
b) applying the at least one anchoring layer (5) onto at least one transition region (7) of the abluminal layer (3), preferably by means of spraying and/or pipetting,
c) applying the at least one anchoring layer (5) such that the at least one anchoring layer (5) extends into the luminal region of the endoprosthesis (10).

10. The method according to claim 9, **characterised in that** the at least one anchoring layer (5) is degradable.

11. The method according to any one of the claims 9 to 10, **characterised in that** the anchoring layer (5, 5a, 5b) is applied such that n abluminal layers (3, 3a) are disposed in the transition region (7) between two of the n+1 anchoring layers (5, 5a, 5b), with n>=1 and n being an integer.

12. The method according to any one of claims 9 to 11, **characterised in that** a further coating (9) is disposed on at least part of the surface of the outer anchoring layer (5, 5b), the further coating preferably containing an endothelial catcher and/or an endothelialisation promoter and/or comprising an endothelialisation-promoting surface.

## Revendications

1. Endoprothèse intraluminale dotée d'un corps et d'au moins une couche abluminale (3) disposée sur le corps, laquelle contient de préférence au moins une substance pharmaceutiquement active, et laquelle est fixée de manière complémentaire dans au moins une zone de transition (7) qui représente une zone d'extrémité de la couche abluminale (3), laquelle est disposée le plus loin dans la direction luminale, au moyen d'au moins une couche d'ancrage (5) disposée sur la couche abluminale (3), sur le corps d'implant, où la couche d'ancrage (5) recouvre simplement la couche abluminale (3) dans la zone de transition,
**caractérisée en ce que** l'au moins une couche d'ancrage (5) s'étend dans la zone luminale de l'endoprothèse (10).

2. Endoprothèse selon la revendication 1, **caractérisée en ce que** la surface de la couche abluminale recouverte par la couche d'ancrage représente dans la zone de transition moins de 30 % de la surface totale de la couche abluminale.

3. Endoprothèse selon l'une des revendications précédentes, **caractérisée en ce que** l'au moins une couche d'ancrage (5) est dégradable.

4. Endoprothèse selon l'une des revendications précédentes, **caractérisée en ce que** la couche abluminale (3) est dégradable.

5. Endoprothèse selon l'une des revendications précédentes, **caractérisée en ce qu'**une liaison par la matière et/ou une liaison par la forme est mise en place dans la zone de transition (7) entre l'au moins une couche abluminale (3) et l'au moins une couche d'ancrage (5).

6. Endoprothèse selon l'une des revendications précédentes, **caractérisée en ce que** n couches abluminales (3, 3a) sont disposées dans la zone de transition (7) entre deux des n + 1 couches d'ancrage (5, 5a, 5b), où n > = 1 et où n est un nombre entier.

7. Endoprothèse selon l'une des revendications précédentes, **caractérisée en ce que** l'au moins une couche d'ancrage (5) est conçue de manière à ce qu'elle se dégrade in vivo plus rapidement que l'au moins une couche abluminale (3).

8. Endoprothèse selon l'une des revendications précédentes, **caractérisée en ce qu'**un nouveau revêtement (9) est disposé au moins partiellement sur la surface de la couche d'ancrage (5, 5b) se situant vers l'extérieur, lequel revêtement contient de préférence un capteur d'endothélium et/ou un promoteur d'endothélialisation, et/ou présente une surface favorisant l'endothélialisation.

9. Procédé de fabrication d'une endoprothèse intraluminale dotée d'un corps et d'au moins une couche abluminale (3) disposée sur le corps, laquelle contient de préférence au moins une substance pharmaceutiquement active, comprenant les étapes suivantes :
a) la mise en place du corps de l'endoprothèse (10),
b) le dépôt de l'au moins une couche d'ancrage (5) sur au moins une zone de transition (7) de la couche abluminale (3), de préférence au moyen d'une pulvérisation et/ou d'un pipetage,
c) le dépôt de l'au moins une couche d'ancrage (5) de telle manière que l'au moins une couche d'ancrage (5) s'étend dans la zone luminale de l'endoprothèse (10).

10. Procédé selon la revendication 9, **caractérisé en ce que** l'au moins une couche d'ancrage (5) est dégradable.

11. Procédé selon l'une des revendications 9 à 10, **caractérisé en ce que** la couche d'ancrage (5, 5a, 5b) est déposée de telle manière que n couches abluminales (3, 3a) sont disposées dans la zone de transition (7) entre deux des n + 1 couches d'ancrage (5, 5a, 5b), où n > = 1 et où n est un nombre entier.

12. Procédé selon l'une des revendications 9 à 11, **caractérisé en ce qu'**un nouveau revêtement (9) est disposé au moins partiellement sur la surface de la couche d'ancrage (5, 5b) se situant vers l'extérieur, lequel revêtement contient de préférence un capteur d'endothélium et/ou un promoteur d'endothélialisation, et/ou présente une surface favorisant l'endothélialisation.
